Europäisches Patentamt

European Patent Office

Office européen des brevets

(11) Numéro de publication: **0 207 873 B1**

## FASCICULE DE BREVET EUROPEEN

(45) Date de publication du fascicule du brevet:
20.12.89

(51) Int. Cl.⁴: **A61F 2/46**

(21) Numéro de dépôt: **86460012.7**

(22) Date de dépôt: **18.06.86**

(54) Instrument porte-prothèse.

(30) Priorité: **24.06.85 FR 8509680**

(43) Date de publication de la demande:
**07.01.87 Bulletin 87/2**

(45) Mention de la délivrance du brevet:
**20.12.89 Bulletin 89/51**

(84) Etats contractants désignés:
**BE CH DE GB IT LI NL**

(56) Documents cités:
**DE-B- 2 101 002**
**FR-A- 2 151 863**
**US-A- 3 067 740**
**US-A- 3 857 389**
**US-A- 4 222 382**

(73) Titulaire: **EQUIPEMENT MEDICAL ET CHIRURGICAL DE L'OUEST, B.P. 15 Boulevard Nominoe, F-35740 Pace(FR)**

(72) Inventeur: **Paul, Frédérick, Marc, Auguste, 31, rue Jean Guéhenno, F-35000 Rennes(FR)**

(74) Mandataire: **Martin, Jean-Jacques et al, Cabinet REGIMBEAU Corre, Martin, Schrimpf, Warcoin, Ahner 11, rue Franz Heller, F-35700 Rennes(FR)**

## Description

La présente invention concerne un instrument porte-prothèse pour prothèse fémorale.

On connaît des instruments porte-prothèse pour prothèse fémorale qui comprennent un tube dans lequel est monté coaxialement un outil impacteur apte à être déplacé axialement par vissage dans ce tube, et un manchon dont l'axe forme un angel aigu avec l'axe du tube. Cet angle est supplémentaire de l'angle que forme l'axe longitudinal de la prothèse avec l'axe de sa tête. La forme de la paroi intérieure du manchon correspond à celle de la tête de la prothèse qui doit être mise en place.

Pour utiliser cet instrument, on emboîte la tête de prothèse dans le manchon, et on visse l'outil impacteur dans le tube jusqu'à ce que son extrémité inférieure vienne s'appuyer contre la face dorsale de la prothèse ou, plus précisément, dans une empreinte prévue à cet effet ménagée dans celle-ci (et couramment appelée "trou d'impact").

On réalise ainsi une solidarisation parfaite de l'instrument avec la prothèse, ce qui permet au chirurgien de la manipuler convenablement en vue de sa mise en place dans le canal médullaire de l'extrémité proximale du fémur. Lorsque l'instrument est solidarisé avec la prothèse, l'axe du tube - et celui de l'outil impacteur - coïncident avec l'axe longitudinal de la prothèse. L'enfoncement de la prothèse dans le fémur est réalisé en frappant sur l'extrémité supérieure, pourvue d'une tête, de l'outil impacteur, au moyen d'un marteau.

Après mise en place de la prothèse dans le fémur, on dévisse l'outil impacteur, ce qui permet de retirer l'instrument.

Ce type d'instrument est généralement apprécié du chirurgien, car il permet un solidarisation rapide et efficace de la prothèse avec l'instrument et un maniement aisé de l'ensemble. Il présente cependant l'inconvénient de ne pouvoir s'adapter qu'à une seule catégorie de prothèses, dont la forme, les dimensions, et l'emplacement du trou d'impact sont bien définis. Dès que l'une de ces caractéristiques est modifiée, il est nécessaire d'utiliser un autre instrument porte-prothèse. Ceci pose évidemment un problème de stockage et de manipulation des instruments car le bloc opératoire doit posséder en permanence un nombre d'instruments porte-prothèse qui est égal à celui relativement élevé des différentes catégories de prothèses fémorales qu'il a à sa disposition.

L'invention vise à résoudre ce problème en proposant un instrument porte-prothèse du type mentionné qui puisse s'adapter à un nombre élevé de prothèses fémorales de formes et de dimensions différentes.

Ce résultat est atteint, conformément à l'invention, par le fait que l'instrument porte-prothèse est pourvu d'un fourreau amovible et interchangeable qui est monté à coulissement dans le manchon et est adapté pour être emboîté sur la tête de la prothèse.

Il suffit alors d'avoir à sa disposition un certain nombre de manchons, de forme extérieure identique permettant leur coulissement dans le manchon, mais de forme intérieure différente permettant leur adaptation sur différentes têtes de prothèses, pour que l'instrument puisse être solidarisé avec des prothèses différentes.

Dans une forme de réalisation préférentielle de l'invention, le manchon et le fourreau sont tous deux de forme cylindrique, le diamètre de la paroi extérieure du fourreau étant égal au diamètre intérieur du manchon.

Il est possible de donner à la paroi intérieure du fourreau une forme de tronc de cône adaptéepour s'emboîter sur une tête de prothèse tronconique, ou de lui donner une forme hémisphérique adaptée pour s'emboîter sur une tête de prothèse en forme de rotule sphérique.

Le fourreau peut être soitune pièce monobloc, soit une pièce constituée de deux demi-coquilles adaptées pour enserrer la tête de la prothèse.

Selon une première forme de réalisation, l'outil impacteur est une tige cylindrique qui présente une partie filetée vissée dans le tube, l'une des ses extrémités étant conformée pour s'appuyer contre la face dorsale de la prothèse, tandis que son autre extrémité porte une tête de vissage et d'impaction.

Dans une autre forme de réalisation, l'outil impacteur est interchangeable ; dans ce cas, l'outil impacteur est monté coulissant dans le tube, son déplacement étant alors avantageusement assuré par une tige de commande qui est vissée dans ce tube et qui porte une tête de vissage et d'impaction.

D'autres caractéristiques et avantages de l'invention apparaîtront de la description et des dessins annexés qui présentent trois modes de réalisation préférentiels de cette invention.

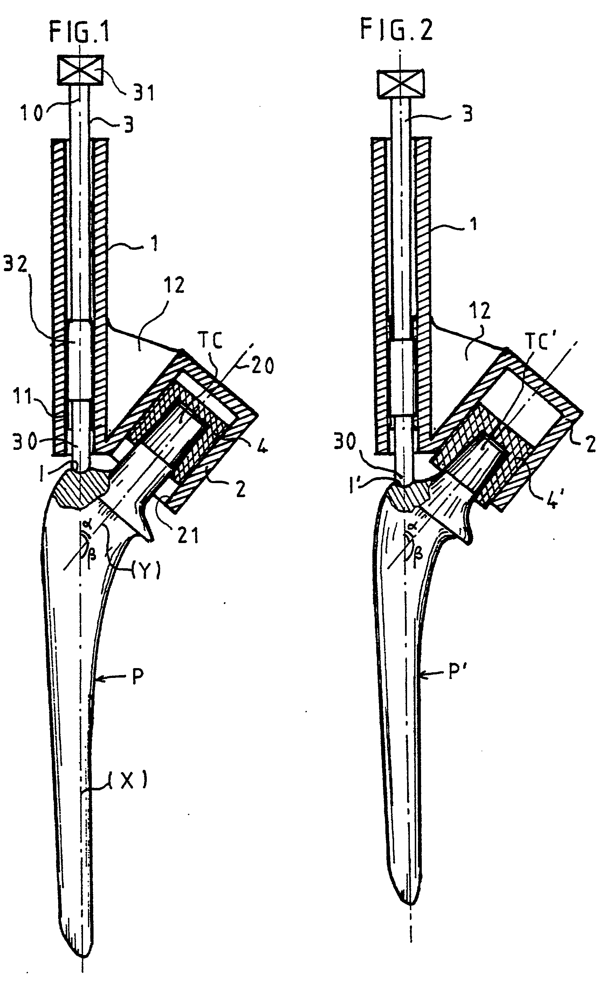

Sur ces dessins, les figures 1 et 2 montrent, en coupe longitudinale, une première forme de réalisation de l'instrument porte-prothèse objet de l'invention, monté sur une prothèse à tête tronconique, respectivement de grande et de petite dimensions.

La figure 3 représente une prothèse à tête sphérique.

La figure 4 est une vue en coupe d'un fourreau en deux parties, adaptable sur la tête sphérique de la prothèse de la figure 3.

La figure 5 est une vue partielle représentant un second mode de réalisation d'un instrument conforme à l'invention, pourvu du fourreau en deux parties de la figure 4, et monté sur la prothèse de la figure 3.

La figure 6 représente, partiellement coupée, une troisième forme de réalisation d'un instrument conforme à l'invention, dont l'outil impacteur est amovible et interchangeable.

Les figures 7, 8 et 9 représentent chacunes en vue de face, un outil impacteur destiné à équiper l'instrument de la figure 6.

Les figures 7a et 8a sont des vues de côté des outils représentés aux figures 7 et 8, respectivement, ces outils étant en appui contre la partie dorsale d'une prothèse.

La figure 9a est une vue en bout de l'outil de la figure 9.

La prothèse représentée à la figure 1 est une endo-prothèse fémorale, de type connu, destinée à être mise en place à la partie proximale d'un fémur détérioré. Cette prothèse P comprend un corps principal allongé, d'axe longitudinal (X), et une tête TC, dont l'axe (Y) forme un angle β avec l'axe longitudinal (X). L'angle β est généralement de l'ordre de 135°. La tête de prothèse TC a la forme d'un tronc de cône, de très faible conicité, qui est destiné à recevoir par emmanchement, de manière bien connue, une tête d'articulation sphérique ou rotule. Cette rotule rapportée ultérieurement n'a pas été représentée car elle ne concerne pas directement la présente invention.

Dans la face supérieure (proximale) dorsale de la porthèse P est ménagée une petite creusure désignée par I.

L'instrument porte-prothèse est constitué de deux parties principales : un tube cylindrique 1, dont la longueur est relativement grande par rapport à son diamètre, et un manchon cylindrique 2; l'axe 10 du tube 1 et l'axe 20 du manchon 2 sont des axes concourants qui forment entre eux un angle aigu α qui est supplémentaire de l'angle β précédemment défini; dans le cas présent, cet angle est donc égal à 45° environ. Le manchon 2 est ouvert du côté tourné vers l'axe 10. Le tube 1 et le manchon 2 sont fixés l'un à l'autre par des moyens appropriés, par exemple par une nervure 12 ; le tube 1 et le manchon 2 peuvent être réalisés d'une seule pièce moulée ou au contraire être deux pièces séparées fixées l'une à l'autre.

A la partie inférieure du tube 1, celui-ci est pourvu d'un taraudage intérieur 11, dans lequel est vissée une tige 3 qui porte une partie filetée 32 complémentaire du taraudage 11. La partie supérieure de cette tige 3 fait saillie à l'extérieur du tube 1 ; elle est pourvue d'une tête de manoeuvre 31, qui présente par exemple une section carrée. La partie inférieure 30 de la tige 3 fait saillie au dessous du tube 1 ; elle est conformée de manière à pouvoir se loger précisément dans l'empreinte (ou creusure) I.

Conformément à l'invention, cet instrument est équipée d'un fourreau amovible et interchangeable 4. Ce fourreau a également la forme d'un manchon, dont la paroi extérieure cylindrique a un diamètre égal (ou très légèrement inférieur) au diamètre de la paroi intérieure 21 du manchon 2, de sorte que le coulissement du fourreau dans le manchon est possible. La paroi intérieure du fourreau 4 a une forme tronconique identique à celle de la tête TC.

A chaque prothèse est associé un fourreau 4 dont la forme intérieure correspond à celle de la tête de prothèse.

Lorsque l'opérateur - en l'occurence le chirurgien ou son assistant - a sélectionné la prothèse qui convient à l'intervention chirurgicale en cours, il sélectionne le fourreau 4 correspondant, coiffe de celui-ci la tête de prothèse par simple emmanchement, et introduit la tête de prothèse coiffée du fourreau dans le manchon 2 ; ensuite, il fait coulisser cet ensemble dans le manchon 2 de telle manière que l'axe longitudinal (X) de la prothèse coïncide approximativement avec l'axe 10 du tube 1 (et de la tige 3 logée dans ce tube); enfin, il visse la tige 3 au moyen d'un outil approprié s'engageant dans la tête de manoeuvre 31, de manière à faire descendre l'extrémité 30 de la tige 3 et la faire venir en appui dans l'empreinte I. Du fait que la forme convexe de cette extrémité de tige 30 est complémentaire de la forme de l'empreinte I, on obtient un centrage parfait de la tige 3 suivant l'axe (X) de la prothèse ; ce centrage se fait automatiquement, grâce à la liberté de coulissement du fourreau 4 à l'intérieur du manchon 2. Lorsque la tige 3 a été suffisamment vissée, on obtient un appui ferme de l'extrémité 30 contre la prothèse P et une solidarisation parfaite de cette dernière avec l'instrument.

On a représenté à la figure 2 une prothèse P' de dimensions plus faibles que la prothèse P de la figure 1, mais d'angle β identique à celui de cette dernière. La prothèse P' possède une tête conique TC' tronconique plus petite que la tête TC de la prothèse P ; en outre, la distance de l'empreinte I' à la tête TC' est sensiblement plus faible que la distance de l'empreinte I à la tête TC. La figure 2 montre qu'il est pourtant possible d'utiliser, pour cette prothèse P', l'instrument de la figure 1, en équipant celui-ce d'un nouveau fourreau 4' dont le diamètre extérieur est le même que celui du manchon 4, mais dont la forme de paroi intérieure est adaptée à la tête TC'. On constate bien entendu que l'amplitude d'enfoncement des fourreaux 4 et 4' dans le manchon 2 et le degré de vissage de la tige 3 dans le tube 1, sont différents pour les prothèses P et P'.

Bien que sur les figures on ait prévu que le fourreau 2 soit fermé à sa partie supérieure, ce manchon pourrait être un tube ouvert à ses deux extrémités ; il pourrait aussi être fermé mais présenter un orifice d'échappement d'air destiné à faciliter le coulissement du fourreau.

Les différentes parties de l'instrument sont réalisées de préférence en métal, par exemple en acier inoxydable ou en alliage léger, tandis que le manchon 4 est réalisé en matière plastique présentant un faible coefficient de frottement, par exemple en polytétrafluoréthylène.

La tête carrée 31, qui sert au vissage de la tige 3 en vue de la fixation de l'instrument sur la prothèse, sert aussi de surface d'impact pour un marteau, qui permet d'enfoncer la prothèse dans le canal médullaire du fémur traité.

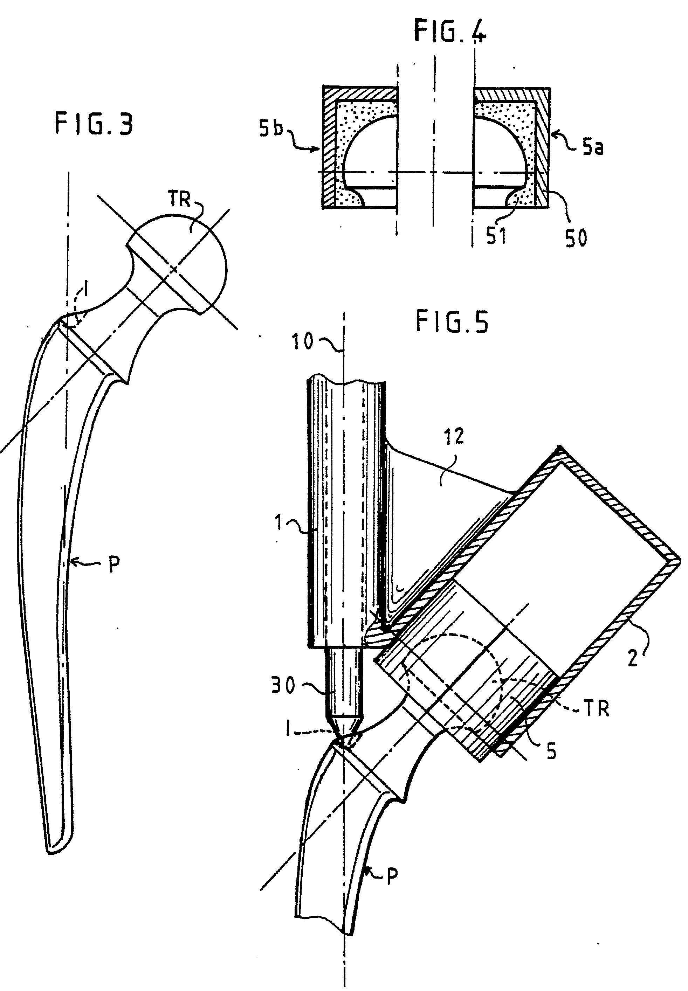

La prothèse représentée à la figure 3 est une prothèse analogue à celle des figures 1 et 2, à la différence qu'elle possède une tête sphérique TR constituant une rotule qui forme partie intégrante du reste de la prothèse.

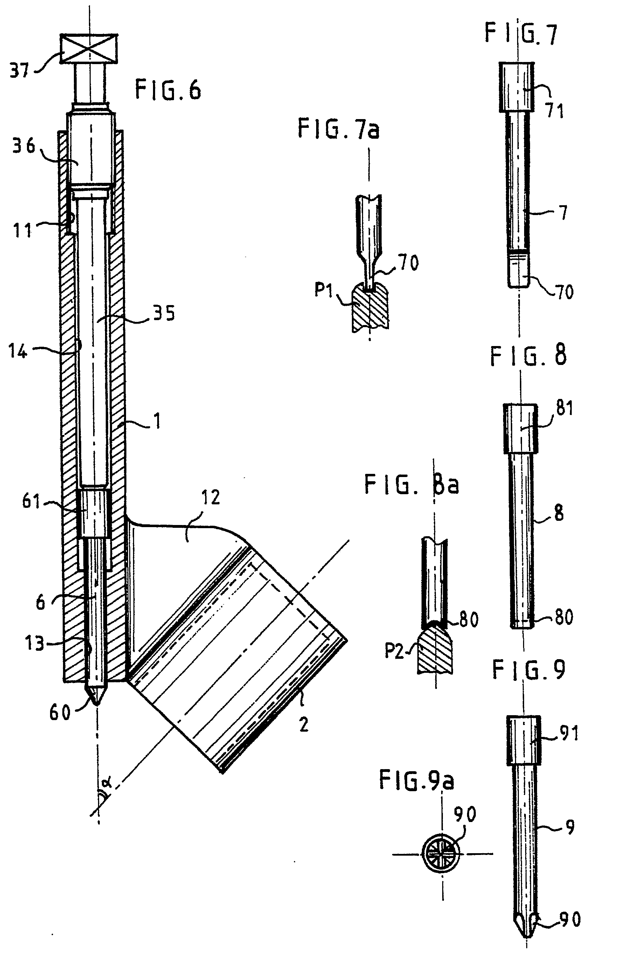

L'instrument porte-prothèse représenté partiellement à la figure 5 est identique à celui des figures 1 et 2, le manchon 2 étant toutefois d'un plus grand diamètre.

Le fourreau amovible et interchangeable équipant cet instrument, désigné par la référence 5, est un fourreau en deux parties ; ces deux parties en forme de demi-coquilles 5a, 5b comprennent chacune une enveloppe extérieure 50 rigide et une garniture interne 51 en matériau souple. L'enveloppe 50 a une forme cylindrique de même diamètre que le diamètre intérieur du manchon 2, ce qui autorise son coulissement dans ce dernier ; le revêtement intérieur 51 a une forme complémentaire de celle de la rotule TR ; le matériau souple utilisé est par exemple un caoutchouc souple ou un matériau mousse du genre polystyrène expansé ; la souplesse de ce revêtement 51 a pour but d'éviter une détérioration superficielle, par exemple un rayage de la tête sphérique TR dont l'état de surface doit être parfaitement lisse.

Comme pour la première forme de réalisation précédemment décrite, il est prévu une série de fourreaux 5 qui présentent la même forme extérieure, mais dont les rayons des cavités hémisphériques intérieures sont différents et peuvent s'adapter aux diverses dimensions de têtes sphériques TR des prothèses que le bloc chirurgical a à sa disposition.

Pour utiliser cet instrument, on emboîte les deux demi-coquilles qui ont été sélectionnées 5a, 5b sur la tête TR de la prothèse qui va être utilisée, on introduit le fourreau 5 ainsi constitué (et la tête TR emprisonnée dans celui-ci) à l'intérieur du manchon 2 et on fait descendre par vissage l'extrémité 30 de l'outil impacteur pour que celui-ci vienne en appui dans l'empreinte I de la prothèse.

L'instrument porte-prothèse qui fait l'objet de la troisième forme de réalisation représentée à la figure 6 a la même forme générale que celle des deux modes de réalisation précédemment décrits. Cet instrument comprend un tube allongé 1 qui est relié à un manchon cylindrique 2 par l'intermédiaire d'une nervure diamètrale 12, l'axe du tube et l'axe du manchon formant entre eux an angle aigu α.

Le tube 1 présente un taraudage 11 réalisé à sa partie supérieure, dans lequel est vissée unt tige de commande 35 présentant une partie filetée 36 et une tête carrée 37. La paroi intérieure du tube 1 présente un premier alésage 14 dans lequel est logée la tige 35 et un second alésage 13, de plus petit diamètre, situé à la partie inférieure de ce tube 1. L'instrument est équipé d'une série d'outils amovibles interchangeables. L'outil 6 représenté en place dans l'instrument à la figure 6 est constitué d'une tige cylindrique qui est montée pour coulisser dans l'alésage intérieur 13 d'une tête plus large supérieure 61 située dans l'alésage 14, et d'une pointe 60 faisant saillie au dessous du tube 1. En vissant la tige 35 dans le tube 1, on commande le déplacement vers le bas de l'outil 6.

Cet outil 6 possède une pointe conique 60 qui est adaptée pour se loger dans une empreinte conique de forme complémentaire ménagée sur la face dorsale de la prothèse, laquelle n'a pas été représentée dans un but de simplification ; l'instrument est pourvu, comme dans les deux modes de réalisation précédents, d'un fourreau apte à coulisser dans le manchon 2 ; ce fourreau peut être indifféremment de type monobloc ou du type constitué de deux demi-coquilles ; il peut être indifféremment prévu pour s'adapter sur des têtes tronconiques ou sphériques. C'est pourquoi ce fourreau n'a pas non plus été représenté à la figure 6.

Cet instrument est utilisé de la même manière que les deux instruments précédemment décrits ; ils présentent toutefois la particularité qu'il est possible de changer l'outil impacteur 6 afin d'adapter l'instrument aux différentes empreintes d'impact prévues dans les prothèses.

L'outil impacteur 7, représenté sur les figures 7 et 7a,a la même forme générale que l'outil 6 qui vient d'être décrit ; il possède unt tête 71 et une pointe 70 ; cette dernière à la forme d'une lame de tournevis qui est adaptéepour se loger dans une rainure ménagée sur la face dorsale de la prothèse P₁(voir figure 7a).

L'outil 8, représenté à la figure 8, possède une tête 81 et une extrémité 80 concave qui est adaptée pour venir en appui contre la face dorsale bombée d'une prothèse P₂ dépourvue d'une empreinte d'impaction (voir figure 8a).

L'outil impacteur 9, représenté à la figure 9, possède une tête 91 et une pointe 90 analogue à celle d'un tournevis cruciforme. Cet outil est adapté pour pénétrer dans un empreinte cruciforme de forme complémentaire.

Pour changer l'outil impacteur, il suffit de dévisser complètement la tige de commande 35 et d'extraire celle-ci du tube 1 ; en retournant l'insturment, l'outil ressort également par gravité de ce tube ; on sélectionne alors l'outil dont la pointe correspond à la forme de l'empreinte de la prothèse considérée - ou de la forme de la face dorsale de cette prothèse, si elle est dépourvue d'un telle empreinte - on l'introduit dans le tube 1, et on visse dans celle-ci la tige 35.

Il va de soi que l'invention n'est pas limitée aux mode de réalisation préférentiels qui viennent d'être décrits à simple titre d'exemples ; elle en englobe au contraire toutes les variantes.

C'est ainsi que la valeur de l'angle α que forment entre eux les axes du tube 1 et du manchon 2 pourrait être différente de la valeur indiquée dans ces exemples ; il suffit que cet angle soit supplémentaire de l'angle β de la prothèse qui doit être utilisée.

Il va de soi qu'on pourrait prévoir d'utiliser des fourreaux en deux parties (demi-coquilles) pour s'adapter sur des têtes de prothèses tronconiques; inversement, on pourrait utiliser des fourreaux monoblocs s'adaptant sur des rotules sphériques; dans ce but, on pourrait prévoir des fourreaux présentant certaines parties élastiques, ce qui permettrait de les adapter sur les têtes de prothèse par encliquetage élastique.

**Revendications**

1. Instrument porte-prothèse, qui comprend un tube (1) dans lequel est monté un outil impacteur (3) apte à être déplacé axialement de manière à venir en appui contre la face dorsale de la prothèse (P) et un manchon (2) dont l'axe forme un angle aigu (α) avec l'axe du tube (1), caractérisé en ce qu'il est pourvu d'un fourreau amovible et interchangeable (4 ; 5) qui est monté à coulissement dans ledit manchon (2) et est adapté pour être emboîté sur la tête (TC ; TH) de la prothèse (P).

2. Instrument porte-prothèse selon la revendication 1, caractérisé en ce que le manchon (2) et le fourreau (4 ; 5) sont de forme cylindrique, le diamètre de la paroi extérieure du fourreau (4 ; 5) étant égal au diamètre intérieur du manchon (2).

3. Instrument porte-prothèse selon l'une des revendications 1 ou 2, caractérisé en ce que la paroi intérieure de fourreau (4) a une forme de tronc de cône adaptée pour s'emboîter sur une tête de prothèse tronconique (TC).

4. Instrument port-prothèse selon l'une des revendications 1 ou 2, caractérisé en ce que la paroi intérieure du fourreau (5) a une forme hémisphérique adaptée pour s'emboîter sur une tête de prothèse en forme de rotule (TR).

5. Instrument porte-prothèse selon l'une des revendications 1 à 4, caractérisé en ce que le fourreau (4) est une pièce monobloc.

6. Instrument porte-prothèse selon l'une des revendications 1 à 4, caractérisé en ce que le fourreau (5) est constitué de deux demi-coquilles (5a, 5b).

7. Instrument porte-prothèse selon l'une des revendications 1 à 6, caractérisé en ce que l'outil impacteur (3) est une tige cylindrique qui présente une partie filetée (32) vissée dans le tube (1), l'une (30) de ses extrémités étant conformée pour s'appuyer contre la face dorsale de la prothèse (P), tandis que son autre extrémité porte une tête (31) de vissage et d'impaction.

8. Instrument porte-prothèse selon l'une des revendications 1 à 6, caractérisé en ce que l'outil impacteur (6, 7, 8, 9) est interchangeable.

9. Instrument porte-prothèse selon la revendication 8, caractérisé en ce que l'outil impacteur (6, 7, 8, 9) est monté coulissant dans le tube (1), son déplacement dans celui-ci étant assuré par une tige de commande (35) qui est vissée dans ce tube (1) et porte une tête (37) de vissage et d'impaction.

10. Instrument porte-prothèse selon l'une des revendications 1 à 9, caractérisé en ce que la paroi intérieure du fourreau (5) est garnie d'un matériau souple (51).

**Claims**

1. Prosthesis holder, which comprises a tube (1) in which there is mounted an impactor tool (3) suitable for axial displacement so as to come to bear against the dorsal face of the prosthesis (P) and a sleeve (2) whereof the axis forms an acute angle ($\alpha$) with the axis of the tube (1), characterized in that it is provided with a removable and interchangeable sliding bush (4; 5) which is mounted to slide in the said sleeve (2) and is adapted to fit on the head (TC; TH) of the prosthesis (P).

2. Prosthesis holder according to Claim 1, characterized in that the sleeve (2) and the sliding bush (4; 5) are cylindrical in shape, the diameter of the external wall of the sliding bush (4; 5) being equal to the internal diameter of the sleeve (2).

3. Prosthesis holder according to either of Claims 1 or 2, characterized in that the internal wall of the sliding bush (4) has a frustoconical shape adapted to fit on a frustoconical prosthesis head (TC).

4. Prosthesis holder according to either of Claims 1 or 2, characterized in that the internal wall of the sliding bush (5) has a hemispherical shape adapted to fit on a ball-shaped prosthesis head (TR).

5. Prosthesis holder according to one of Claims 1 to 4, characterized in that the sliding bush (4) is in a single piece.

6. Prosthesis holder according to one of Claims 1 to 4, characterized in that the sliding bush (5) is composed of two half moulds (5a, 5b).

7. Prosthesis holder according to one of Claims 1 to 6, characterized in that the impactor tool (3) is a cylindrical rod which has a threaded part (32) screwed into the tube (1), one (30) of its ends being shaped to bear against the dorsal face of the prosthesis (P), while its other end carries a screwing and an impaction head (31).

8. Prosthesis holder according to one of Claims 1 to 6, characterized in that the impactor tool (6, 7, 8, 9) is interchangeable.

9. Prosthesis holder according to Claim 8, characterized in that the impactor tool (6, 7, 8, 9) is mounted sliding in the tube (1), its displacement therein being ensured by a control rod (35) which is screwed into this tube (1) and carries a screwing and impaction head (37).

10. Prosthesis holder according to one of Claims 1 to 9, characterized in that the internal wall of the sliding bush (5) is lined with a flexible material (51).

**Patentansprüche**

1. Prothesenhalter, der einen Tubus (1), in den eine Stoßvorrichtung (3) montiert ist, die geeignet ist, axial in einer solchen Weise bewegt zu werden, daß sie sich auf das Rückenteil der Prothese (P) stützt, und eine Manschette (2) aufweist, deren Achse einen spitzen Winkel ($\alpha$) mit der Achse des Tubus bildet, dadurch gekennzeichnet, daß er mit einem abnehmbaren und austauschbaren Futteral (4; 5) versehen ist, das gleitend in der Manschette (2) befestigt ist und geeignet ist, genau auf den Kopf (TC; TH) der Prothese aufgepaßt zu werden.

2. Prothesenhalter nach Anspruch 1, dadurch gekennzeichnet, daß die Manschette (2) und das Futteral (4; 5) eine zylindrische Form aufweisen, wobei der Durchmesser der äußeren Seitenwand des Futterals (4; 5) gleich ist dem inneren Durchmesser der Manschette.

3. Prothesenhalter nach Anspruch 1 oder 2, dadurch gekennzeichnet, daß die innere Seitenwand des Futterals (4) eine Kegelstumpfform aufweist, die geeignet ist, auf einen kegelstumpfartigen Prothesenkopf (TC) aufgepaßt zu werden.

4. Prothesenhalter nach Anspruch 1 oder 2, dadurch gekennzeichnet, daß die innere Seitenwand des Futterals (5) eine halbkugelige Form aufweist, die geeignet ist, auf einen kugelgelenkförmigen Prothesenkopf (TR) aufgepaßt zu werden.

5. Prothesenhalter nach einem der Ansprüche 1 bis 4, dadurch gekennzeichnet, daß das Futteral (4) aus einem Block besteht.

6. Prothesenhalter nach einem der Ansprüche 1 bis 4, dadurch gekennzeichnet, daß das Futteral (5) aus zwei Halbschalen (5a, 5b) besteht.

7. Prothesenhalter nach einem der Ansprüche 1 bis 6, dadurch gekennzeichnet, daß die Stoßvorrichtung (3) ein zylindrischer Schaft ist, der einen in den Tubus (1) geschraubten Teil (32) aufweist, wobei eines ihrer Außenteile ausgebildet ist, um sich auf das Rückenteil der Prothese zu stützen, während ihr anderes Außenteil einen Schraub- und Stoßkopf (31) aufweist.

8. Prothesenhalter nach einem der Ansprüche 1 bis 6, dadurch gekennzeichnet, daß die Stoßvorrichtung (6, 7, 8, 9) auswechselbar ist.

9. Prothesenhalter nach Anspruch 8, dadurch gekennzeichnet, daß die Stoßvorrichtung (6, 7, 8, 9) gleitend in den Tubus (1) montiert ist, wobei ihre Bewegung in demselben durch einen Steuerschaft (35), der in den Tubus (1) geschraubt ist und einen Schraub- und Stoßkopf (37) aufweist, sichergestellt wird.

10. Prothesenhalter nach einem der Ansprüche 1 bis 9, dadurch gekennzeichnet, daß die innere Seitenwand des Futterals (5) mit einem flexiblen Material versehen ist.

FIG.1

FIG.2

FIG. 4

FIG. 3

FIG. 5

FIG.6

FIG.7

FIG.7a

FIG.8

FIG.8a

FIG.9

FIG.9a